(19) Europäisches Patentamt
European Patent Office
Office européen des brevets

(11) Veröffentlichungsnummer: **0 158 083**
**A2**

(12) # EUROPÄISCHE PATENTANMELDUNG

(21) Anmeldenummer: 85102092.5

(22) Anmeldetag: 26.02.85

(51) Int. Cl.[4]: **A 61 K 31/42**
A 61 K 31/425, C 07 D 263/58
C 07 D 277/82

(30) Priorität: 12.04.84 DE 3413876

(43) Veröffentlichungstag der Anmeldung: 16.10.85 Patentblatt 85/42

(84) Benannte Vertragsstaaten: AT BE CH DE FR GB IT LI LU NL SE

(71) Anmelder: LUDWIG HEUMANN & CO GMBH
Heideloffstrasse 18-28 Postfach 2260
D-8500 Nürnberg(DE)

(72) Erfinder: Mörsdorf, Peter, Dr. Dipl.-Chem.
Untere Bahnhofstrasse 16
D-8501 Cadolzburg(DE)

(72) Erfinder: Schickaneder, Helmut, Dr. Dipl.-Chem.
Moosäcker 25
D-8501 Eckental(DE)

(72) Erfinder: Engler, Heidrun, Dr.
Ringstrasse 23
D-8501 Cadolzburg(DE)

(72) Erfinder: Szelenyi, Istan, Dr.
Brahmsstrasse 16
D-8501 Schwaig(DE)

(72) Erfinder: Ahrens, Kurt Henning, Dr.
Praterstrasse 9
D-8500 Nürnberg(DE)

(72) Erfinder: Brune, Kay, Prof. Dr.
Am Weiheracker 17
D-8525 Marloffstein(DE)

(74) Vertreter: Kraus, Walter, Dr. et al,
Patentanwälte Kraus Weisert & Partner
Thomas-Wimmer-Ring 15
D-8000 München 22(DE)

(54) **Arzneimittel.**

(57) Es werden Arzneimittel mit antiphlogistischer Wirkung beschrieben, welche als Wirkstoff ein Benzazolderivat der allgemeinen tautomeren Formeln I

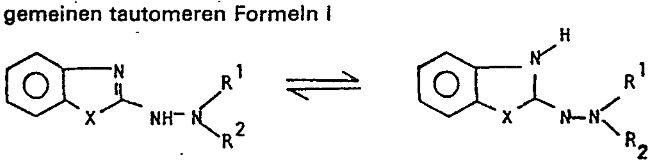

(I)

in denen X für ein Schwefel- oder Sauerstoffatom steht, R[1] ein Wasserstoffatom bedeutet und R[2] für eine Hydroxymethyl-, Formyl-, Carbniedrigalkoxy- oder eine Niedrigacylgruppe steht oder R[1] und R[2] zusammen für die Gruppierung

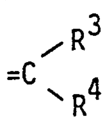

stehen, wobei R[3] und R[4] unabhängig voneinander ein Wasserstoffatom oder eine $C_1$-$C_6$-Alkylgruppe bedeuten, oder ein physiologisch annehmbares Salz davon enthalten. Diese Arzneimittel sind insbesondere zur Bekämpfung entzündlicher Erkrankungen sowie zur Inhibierung des Lipoxygenase- und/oder Cyclooxygenasewegs des Arachidonsäurestoffwechsels geeignet.

EP 0 158 083 A2

Croydon Printing Company Ltd

# Arzneimittel

Die Erfindung betrifft ein Arzneimittel mit hoher antiinflammatorischer Wirkung. Die Erfindung betrifft weiterhin die Verwendung bestimmter Benzazolderivate zur Herstellung eines Arzneimittels mit hoher antiinflammatorischer Wirkung sowie die Verwendung dieser Benzazolderivate zur Bekämpfung von entzündlichen Erkrankungen. Die Verbindungen der allgemeinen Formel I sind zum Teil (X = S) bereits in der japanischen Offenlegungsschrift Nr. 76 35 428 (1976) beschrieben. Die Herstellung dieser Verbindungen kann durch eine chemische Kondensationsreaktion erfolgen, bei der äquimolare Lösungen von einem 2-Hydrazino-Benzazolderivat mit Aldehyden oder Ketonen in Essigsäure vier Stunden bei 50°C umgesetzt werden. Nach dem Einengen der Lösung wird das Benzazolderivat der allgemeinen Formel I durch Kristallisation analysenrein gewonnen. Weiterhin werden in der japanischen Offenlegungsschrift Nr. 76 35 428 (1976) Benzothiazolverbindungen enthaltende Schädlingsbekämpfungsmittel beschrieben. Hinweise auf etwaige arzneiliche Wirkungen der dort beschriebenen Benzazolverbindungen fehlen in dieser Druckschrift.

Die Synthese zur Herstellung von Benzazolderivaten (X = S), bei denen $R^1$ für ein Wasserstoffatom und $R^2$ für eine Formylgruppe steht, ist in den DE-OS'en 27 03 280 bzw. 27 27 924 beschrieben. Aus diesen Ver-Verbindungen sind ohne weiteres durch Reduktion mit einem metallorganischen Reduktionsmittel, zum Beispiel Lithiumalanat, in einem inerten Lösungsmittel, wie Tetrahydrofuran, die entsprechenden Verbindungen erhältlich, in denen $R^2$ eine Hydroxymethylgruppe darstellt. Die Herstellung dieser Benzothiazolverbindungen kann auch auf folgende Weise durchgeführt werden: Äquimolare Lösungen von 2-Hydrazinobenzothiazol und Formamid in Essigsäure werden fünf Stunden auf 80°C erwärmt. Die Reinigung des Produkts erfolgt durch Kristallisation.

Weiter werden gemäß den erwähnten Offenlegungsschriften Benzothiazolderivate zur Durchführung von Vinylchlorid-Polymerisationen verwendet; Hinweise auf etwaige arzneiliche Wirkungen der dort beschriebenen Benzothiazolderivate fehlen in diesen Druckschriften.

Die Herstellung von Benzothiazolderivaten, bei denen $R^1$ für ein Wasserstoffatom und $R^2$ für eine Acylgruppe steht, ist in der DE-OS 22 50 077 beschrieben. In dieser Druckschrift werden Benzazolderivate enthaltende Schädlingsbekämpfungsmittel erwähnt. Arzneiliche Wirkungen dieser Verbindungen sind nicht beschrieben. In dazu analoger Weise sind Verbindungen erhältlich, bei denen X ein Sauerstoffatom ist.

Die Herstellung von Benzazolderivaten, bei denen X für ein Schwefel- oder Sauerstoffatom, $R^1$ für ein Wasserstoffatom und $R^2$ für eine Carbalkoxygruppe stehen, erfolgt durch Umsetzung von 2-Chlorbenzazolderivaten mit Hydrazincarbonsäurealkylestern in Ethanol 12 Stunden bei Rückflußtemperatur.

Der Erfindung liegt die Aufgabe zugrunde, ein neues Arzneimittel mit antiphlogistischer Wirkung zur Verfügung zu stellen.

Man weiß heute, daß an der Entstehung von entzündlichen und allergischen Prozessen Arachidonsäuremetaboliten, wie cyclische Endoperoxide, "Slow Reacting Substances of Anaphylaxis"(SRS-A, Leucotriene), Prostaglandine und Thromboxane, beteiligt sind. Diese Metaboliten werden durch die Enzyme Lipoxygenase und Cyclooxygenase gebildet. Es ist daher vorteilhaft, Arzneimittel zu entwickeln, die Wirkstoffe enthalten, die eine umfassendere antiphlogistische Wirkung entwickeln als die bis heute bekannten. Bis heute sind relativ wenig Verbindungen bekannt, die entweder selektiv die Lipoxygenase oder gleichzeitig Lipoxygenase und Cyclooxygenase hemmen. Bekannte Hemmer dieser Art sind zum Beispiel Benoxaprofen und 3-Amino-1-(3-trifluormethylphenyl)-pyrazolin.

Oberraschenderweise hemmen die erfindungsgemäß verwendbaren Benzazolderivate in einem sehr starken Maße zum Teil selektiv das Enzym Lipoxygenase, während einige Benzazolderivate beide Enzyme, Lipoxygenase und Cyclooxygenase, inhibieren. Die erfindungsgemäßen cyclooxygenase- und/oder lipoxygenasehemmenden Benzazolderivate sind somit als Arzneimittel bei der Behandlung von entzündlichen und allergischen Erkrankungen einsetzbar. Beispiele sind die Prophylaxe oder die Behandlung

von arthritischen Krankheitszuständen, Hautentzündungen, Augenentzündungen, Gewebenekrosen, Gewebeabstoßungen nach chirurgischen Transplantationen, des Schmerzes, des Fiebers und des Asthmas.

Gegenstand der Erfindung ist daher ein Arzneimittel mit antiphlogistischer, insbesondere cyclooxygenase- und/oder lipoxygenasehemmender Wirkung, das dadurch gekennzeichnet ist, daß es neben üblichen Hilfs- und Trägerstoffen als Wirkstoff ein Benzazolderivat der allgemeinen tautomeren Formeln I

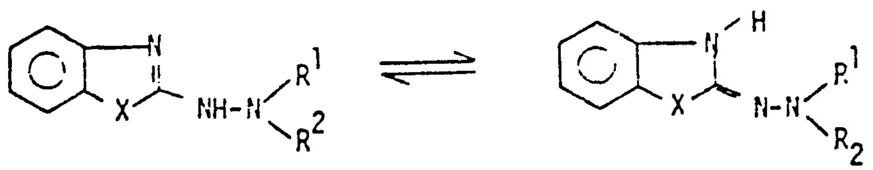

(I)

in denen X für ein Schwefel- oder Sauerstoffatom steht, $R^1$ ein Wasserstoffatom bedeuten und $R^2$ für eine Hydroxymethyl-, Formyl-, Carbniedrigalkoxy- oder eine Niedrigacylgruppe steht oder $R^1$ und $R^2$ zusammen für die Gruppierung

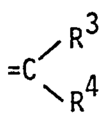 stehen, wobei $R^3$ und $R^4$ unabhängig voneinander ein Wasserstoffatom oder eine $C_1$-$C_6$-Alkylgruppe bedeuten, oder ein physiologisch annehmbares Salz davon enthält. Weitere Gegenstände der Erfindung sind die Verwendung dieser Derivate und ihrer Salze zur Bekämpfung von entzündlichen Erkrankungen bzw. zur Herstellung von pharmazeutischen Zubereitungen mit antiphlogistischer, insbesondere cyclooxygenase- und/oder lipoxygenasehemmender, Wirkung.

In den allgemeinen tautomeren Formeln I bedeutet X ein Schwefel- oder Sauerstoffatom. Die Substituenten $R^1$ und $R^2$ stehen unabhängig voneinander für ein Wasserstoffatom, eine Hydroxymethyl-, Formyl-, Carbniedrigalkoxy- oder eine Niedrigacylgruppe. Hierin wird unter "Niedrigalkoxy" bzw. "Niedrigacyl" eine Gruppe mit 1 bis 6, vorzugsweise 1 bis 3, Kohlenstoffatomen im Alkylteil verstanden. Beispiele für Niedrigalkoxygruppen sind Methoxy-, Ethoxy, n-Propoxy-, i-Propoxy-, n-Butoxy- und t-Butoxygruppen, während Beispiele für Niedrigacylgruppen die Acetyl- und Propionylgruppe sind.

Die Verbindungen, bei denen $R^1$ ein Wasserstoffatom und $R^2$ eine Formyl- oder Carbethoxygruppe bedeuten, werden bevorzugt.

Weiterhin bedeuten $R^1$ und $R^2$ die Gruppierung $=C\genfrac{}{}{0pt}{}{/R^3}{\backslash R^4}$, wobei $R^3$ und $R^4$ unabhängig voneinander für ein Wasserstoffatom oder eine $C_1$-$C_6$-Alkylgruppe, vorzugsweise eine $C_1$-$C_3$-Alkylgruppe, stehen.
Beispiele für derartige Alkylgruppen sind Methyl-, Ethyl-, n-Propyl-, i-Propyl-, n-Butyl-, i-Butyl-, n-Pentyl- und n-Hexylgruppen. Bevorzugt werden Verbindungen, bei denen $R^3$ für ein Wasserstoffatom und $R^4$ für eine Methyl- oder Ethylgruppe stehen oder bei denen $R^3$ und $R^4$ für eine Methylgruppe stehen.

Die erfindungsgemäß verwendete Verbindung wird vorzugsweise oral verabreicht. Gewöhnlich beträgt die orale Tagesdosis etwa 0,05 bis 100 mg/kg, vorzugsweise 0,1 bis 10 mg/kg, Körpergewicht. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß. Im Falle der Applikation größerer Mengen kann es empfehlenswert sein, diese in mehreren Einzelgaben über den Tag zu verteilen.

Für die orale Verabreichung kann der Wirkstoff beispielsweise in Form von Kapseln formuliert werden, die durch herkömmliche Maßnahmen mit pharmazeutisch annehmbaren Exzipitientien, zum Beispiel Bindemitteln (wie vorgelatinisierter Maisstärke, Polyvinylpyrrolidon oder Hydroxypropylmethylcellulose), Füllstoffen (wie Lactose, mikrokristalline Cellulose oder Calciumphosphat), Schmiermitteln (wie Magnesiumstearat, Talk oder Kieselsäure), Sprengmitteln (zum Beispiel Kartoffelstärke oder Natriumstärkeglykolat) oder Befeuchtungsmitteln (zum Beispiel Natriumlaurylsulfat), hergestellt werden.

Flüssige Zubereitungen für die orale Verabreichung oder für ein direktes Einträufeln können beispielsweise die Form von Lösungen, Sirups oder Suspensionen haben, oder sie können als Trockenprodukt zur Rekonstitution mit Wasser oder einem anderen geeigneten Träger vor dem Gebrauch präsentiert werden. Solche flüssigen Zubereitungen können durch herkömmliche Maßnahmen mit pharmazeutisch annehmbaren Additiven, zum Beispiel Suspendierungsmitteln, wie Sorbitsirup, Methylcellulose oder hydrierten eßbaren Fetten, Emulgierungsmitteln, zum Beispiel Lecithin oder Acacia, nichtwäßrigen Trägern, zum Beispiel Mandelöl, öligen Estern oder Ethylalkohol, und Konservierungsmitteln, zum Beispiel Methyl- oder Propylparahydroxybenzoaten oder Sorbinsäure, hergestellt werden.

Für die bukkale Verabreichung können die Zubereitungen in Form von Tabletten oder Lutschpastillen, die auf herkömmliche Weise formuliert werden, vorliegen. Die erfindungsgemäß verwendete Verbindung kann für die parenterale Verabreichung durch Injektion oder für die Infusion formuliert werden. Zubereitungen für die Injektion können in Einheitsdosisform, zum Beispiel in Ampullen, oder in Vieldosenbehältern mit einem zugegebenen Konservierungsstoff vorliegen. Die Zubereitungen können auch solche Formen, wie Suspensionen, Lösungen oder Emulsionen in öligen oder wäßrigen Trägern, einnehmen, und sie können Formulierungsmittel, wie Suspendierungs-, Stabilisierungs- und/oder Dispergierungsmittel, enthalten. Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die folgenden pharmakologischen Untersuchungen der erfindungsgemäßen Verbindungen geben Aufschluß über ihre überraschende antiphlogistische Wirksamkeit.

## 1. Rattenpfoten-Ödem-Test

### 1.1. Methode

Bei männlichen Ratten (100 - 120 g) wurde durch subplantare Injektion von 0,1 ml 2% Carrageenin(in wäßriger 0,9 % NaCl-Lösung gelöst) ein akutes entzündliches Pfotenödem hervorgerufen. Das Pfotenvolumen wurde 1 und 4 Stunden nach der Carrageenin-Injektion mittels eines Pfotenvolumenmeßgerätes gemessen. Es wurden die Differenzen in den Pfotenvolumina zwischen den zwei Meßungen berechnet. Die Prüfsubstanzen wurden in 1%-iger Tylose suspendiert, eine Stunde nach der Carrageenin-Injektion intragastral mittels einer Magenschlundsonde verabreicht.
Die Kontrolltiere erhielten nur den Trägerstoff (1% Tylose).
Die prozentuale Hemmung der Volumenzunahme bei den behandelten Tieren ergab sich durch Vergleich mit den unbehandelten Kontrolltieren. Die mittleren Hemmdosen ($ED_{50}$) wurden mit Hilfe der Regressionskurven berechnet.

### 1.2. $ED_{50}$-Werte

Tabelle 1

| Beispiel-Nr. | $ED_{50}$ (mg/kg) ig |
|---|---|
| 1 | 15 |
| 2 | 10 |
| 3 | 20 |
| 4 | 10 |
| 5 | 20 |
| 6 | 10 |
| 7 | 20 |
| 8 | 14 |

2. Leukozyten-Migrationstest

1. Methode

Bei männlichen Ratten (140 - 150 g) wurde ein mit 2 %-iger Carrageenin-Lösung getränktes Polyester-Schwämmchen im Nackenbereich subcutan implantiert. (vgl. Higgs, G.A. et al. Eur. J. Pharmacol. 66, 81 (1980), Ford-Hutchinson, A.W. et al. J. Pharmacol. Methods 1, 3 (1978)).
24 Stunden später wurden die Schwämmchen entfernt. Das Auswaschen der eingewanderten Leukozyten erfolgte in einer PBS-Lösung (pH 7.4, 0,5 % Trypsin, 10 Einheiten/ml Heparin) bei 37 $^{o}$ C 30 Minuten lang.
Anschließend wurden die Schwämmchen vorsichtig ausgedrückt und zentrifugiert (15 Minuten, 500 rpm). Die Leukozyten in der Waschflüssigkeit wurden dann ausgezählt. Die Prüfsubstanzen, die in 1%-iger Tylose aufgenommen wurden, und das Vehikel (1 %-ige Tylose) wurden unmittelbar nach der Implantation des Schwämmchens, nach 5 und nach 21 Stunden später intragastral mittels einer Schlundsonde appliziert. Die prozentuale Hemmung der Leukozyten-Migration nach Gabe der Prüfsubstanzen wurde im Vergleich zur Kontrolle ermittelt. Die mittleren Hemmdosen ($ED_{50}$) wurden anhand der Regressionsgeraden berechnet.

2.1. Hemmwirkung

Tabelle 2

| Beispiel-Nr. | Dosis (ig) | Hemmung der Leukozyten-migration (Kontrolle = 0%) |
|---|---|---|
| 1 | 40 mg/kg | 50 % |
| 2 | 50 mg/kg | 34 % |
| 8 | 50 mg/kg | 67 % |

## 3. Hemmung der PGE 2- und LTC 4-Synthese und -Freisetzung

### 3.1. Methode

Die in-vitro-Wirksamkeit der erfindungsgemäßen Verbindungen wird nach an sich bekannten Methoden nachgewiesen. (vgl. Brune, K. et al. Nature 274, 262 (1978); Brune, K. et al. Naunyn Schmiedeberg's Arch. Pharmacol. 315, 269 (1981); Peskar, B.A. et al. (1979) in: Radioimmunoassay of drugs and hormones in cardiovascular medicine, Eds. Albertini, A., Da Prada, M., Peskar, B.A., Elsevier, Amsterdam)

### 3.2. Hemmwirkung

Tabelle 3

| Beispiel-Nr. | PGE 2 (% von Kontrollen bei) | | | | LTC 4 (% von Kontrollen bei) | | | |
|---|---|---|---|---|---|---|---|---|
| | $10^{-4}$ | $10^{-5}$ | $10^{-6}$ | $10^{-7}$ | $10^{-4}$ | $10^{-5}$ | $10^{-6}$ | $10^{-7}$ |
| 1 | 12 | 38 | 89 | NG | (2) | > 100 | >100 | NG |
| 2 | 20 | 43 | 103 | NG | 16 | 41 | 89 | NG |
| 3 | 8 | 37 | 61 | NG | 24 | > 100 | >100 | NG |
| 8 | 82 | 112 | 105 | NG | 11 | 70 | >100 | NG |

NG = nicht gemessen

Die übrigen Beispiele zeigen ähnliche pharmakologische Wirkungen.

Beispiel 1

Herstellung von N-(Benzothiazol-2-yl)-acetaldehydhydrazon

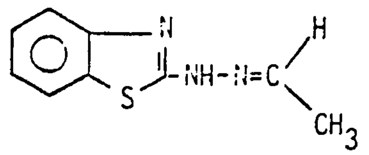

3,3 g (20 mmol) 2-Hydrazinobenzothiazol und 0.88 g (30 mmol) Acetaldehyd werden in 30 ml Essigsäure (98 %) gelöst und 2 h auf 50 °C erwärmt. Nach dem Einengen der Reaktionslösung wird der Rückstand aus Ethanol/Isopropanol umkristallisiert.

Farblose Kristalle vom Schmelzpunkt 200 - 201 °C

Ausbeute: 2,1 g (55,3 % d.Th.)

Rf = 0,69 ($CH_2Cl_2$ / MeOH 9 : 1)

| | | | | |
|---|---|---|---|---|
| $C_9H_9N_3S$ (191) | Ber.: | C 56,52 | H 4,74 | N 21,97 |
| | Gef.: | C 56,62 | H 4,76 | N 21,86 |

$^1$H-NMR-Spektrum: (d$_6$-DMSO, TMS als interner Standard)

$\delta$ = 2.0 (d) (-$CH_3$) 3 H,
6.97 - 7.87 (m) (Aromaten-H, =C-H) 5 H,
11.73 (s) (N-H) (austauschbar mit $D_2O$) 1 H ppm.

Beispiel 2

Herstellung von N-(Benzothiazol-2-yl)acetonhydrazon

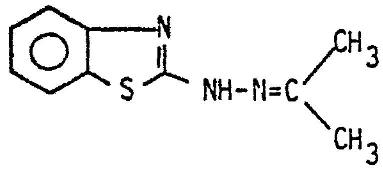

Die Umsetzung erfolgt analog Beispiel 1 mit 2-Hydrazinobenzothiazol und Aceton.

Farblose Kristalle vom Schmelzpunkt 195 - 197 °C

Ausbeute: 2,3 g (56 % d.Th.)

Rf = 0,61 ($CH_2Cl_2$ / MeOH 9:1 )

| | | | | |
|---|---|---|---|---|
| $C_{10}H_{11}N_3S$ (205) | Ber.: | C 58.51 | H 5.40 | N 20.47 |
| | Gef.: | C 59.20 | H 5.68 | N 20.76 |

$^1$H-NMR-Spektrum: ($d_6$-DMSO, TMS als interner Standard)

$\delta$ = 1.93 (s) (2 x $C\underline{H}_3$) 6 H,
6.83 - 7.70 (m) (Aromaten-$\underline{H}$) 4 H,
9.0 (s) (N-$\underline{H}$) (austauschbar mit $D_2O$) 1 H ppm.

Beispiel 3

Herstellung von N-(Benzothiazol-2-yl)-propionaldehydhydrazon

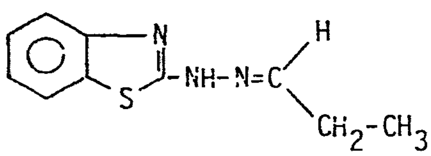

Die Herstellung erfolgt analog Beispiel 1 mit 2-Hydrazinobenzothiazol und Propionaldehyd.

Farblose Kristalle vom Schmelzpunkt 171 - 172 °C

Ausbeute: 2.6 g (63,4 % d.Th.)

Rf = 0,72 ( $CH_2Cl_2$ / MeOH 9:1)

$C_{10}H_{11}N_3S$ (205)

$^1$H-NMR-Spektrum: (d$_6$-DMSO, TMS als interner Standard)

δ = 1.07 (t) (-C$\underline{H}_3$) 3 H,
2.30 (m) (-C$\underline{H}_2$) 2 H,
6.93 - 7.83 (m) (Aromaten-$\underline{H}$, =C$\underline{H}$) 5 H,
11.67 (s) (N-$\underline{H}$) (austauschbar mit $D_2O$) 1 H ppm.

## Beispiel 4

Herstellung von N-(Benzothiazol-2-yl)-methylethylketonhydrazon

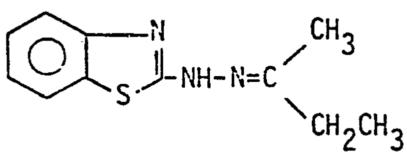

Die Herstellung erfolgt analog Beispiel 1 mit 2-Hydrazinobenzothiazol und Methylethylketon.

Farblose Kristalle vom Schmelzpunkt 123 - 129 °C

Ausbeute: 2,4 g (54,8 % d.Th.)

Rf = 0.73 ($CH_2Cl_2$ / MeOH 9:1)

$^1$H-NMR-Spektrum: (d$_6$-DMSO, TMS als interner Standard)

= 1.1 (t) (-$C\underline{H}_3$) 3 H,
2.0 (s) (-$C\underline{H}_3$) 3 H,
2.33 (q) (-$C\underline{H}_2$) 2 H,
6.97 - 7.83 (m) (Aromaten-$\underline{H}$) 4 H,
11.13 (s) (N-$\underline{H}$) (austauschbar mit $D_2O$) 1 H ppm.

Beispiel 5

Herstellung von N-(Benzoxazol-2-yl)-acetaldehydhydrazon

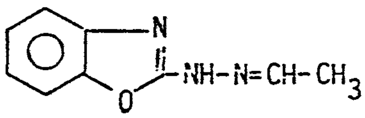

Die Herstellung erfolgt analog Beispiel 1 mit 2-Hydrazinobenzoxazol und Acetaldehyd.

Farblose Kristalle vom Schmelzpunkt 169 - 170 $^{o}$ C

Ausbeute: 1,7 g (48,6 % d.Th.)

Rf = 0,75 ($CH_2Cl_2$ / MeOH 9:1)

$C_9H_9N_3O$ (175)

$^1$H-NMR-Spektrum: (d$_6$-DMSO, TMS als interner Standard)

$\delta$ = 1.93 (d) (-$C\underline{H}_3$) 3 H,
6.93 - 7.67 (m) (Aromaten-$\underline{H}$, =C-$\underline{H}$) 5 H,
11.33 (s, breit) (N-$\underline{H}$) (austauschbar mit $D_2O$) 1 H ppm.

Beispiel 6

Herstellung von N-(Benzoxazol-2-yl)-acetonhydrazon

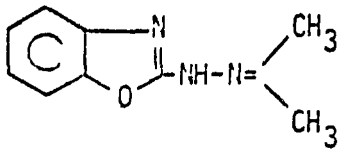

Die Herstellung erfolgt analog Beispiel 1 mit 2-Hydrazinobenzoxazol und Aceton.

Farblose Kristalle vom Schmelzpunkt 157 - 158 °C

Ausbeute: 2,7 g (69,8 % d.Th.)

Rf = 0,74 ($CH_2Cl_2$ / MeOH 9 : 1)

| | |
|---|---|
| $^1$H-NMR-Spektrum: (d$_6$-DMSO, TMS als interner Standard) | $\delta$ = 1.93 (s) (-C$\underline{H}_3$) 3 H,<br>2.07 (s) (-C$\underline{H}_3$) 3 H,<br>6.90 - 7.58 (m) (Aromaten-$\underline{H}$) 4 H,<br>8.67 (s) (N-$\underline{H}$) (austauschbar mit $D_2O$) 1 H ppm. |

Beispiel 7

Herstellung von N-(Benzoxazol-2-yl)propionaldehydhydrazon

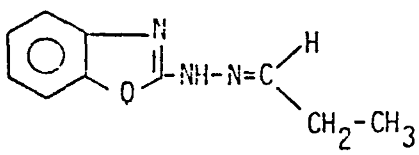

Die Herstellung erfolgt analog Beispiel 1 mit 2-Hydrazinobenzoxazol und Propionaldehyd.

Farblose Kristalle vom Schmelzpunkt 112 - 113 °C

Ausbeute: 1,9 g (50,3 % d.Th.)

Rf = 0,69 ($CH_2Cl_2$ / MeOH 9 : 1)

$^1H$-NMR-Spektrum: ($d_6$-DMSO, TMS als interner Standard)

$\delta$ = 0.95 (t) (-$CH_3$) 3 H,
2.17 (m) (-$CH_2$) 2 H,
6.78 - 7.53 (m) (Aromaten-H) 4 H,
11.47 (s) (N-H) (austauschbar mit $D_2O$) 1 H ppm.

Beispiel 8

Herstellung von Benzothiazol-2-on-(ß-formyl-hydrazon)

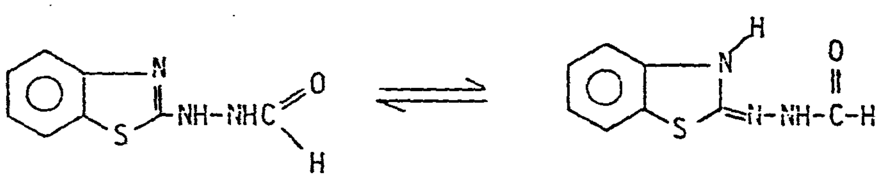

In 10 ml Essigsäure (98 %) werden 3,3 g (20 mmol) 2-Hydrazinobenzothiazol mit 0,9 g (20 mmol) Formamid 5 Stunden bei 80 °C umgesetzt. Der Rückstand wird aus Isopropanol umkristallisiert.

Farblose Kristalle vom Schmelzpunkt 226 °C

Ausbeute: 2,0 g (52,6 % d.Th.)

Rf = 0,50 ($CH_2Cl_2$ / MeOH 9:1)

| | | | | |
|---|---|---|---|---|
| $C_8H_7N_3OS$ (193) | Ber.: | C 49,73 | H 3,65 | N 21,75 |
| | Gef.: | C 50,35 | H 3,76 | N 21,63 |

$^1$H-NMR-Spektrum: (d$_6$-DMSO, TMS als interner Standard)

$\delta$ = 6.93 - 7.87 (m) (Aromaten-H) 4 H,
8.17 (s) (-C(=O)-H) 1 H,
10.17 (s) (2 x N-H) (austauschbar mit $D_2O$) 2 H ppm.

## Beispiel 9

**Herstellung von N$^2$-(Benzothiazol-2-yl)-hydrazincarbonsäureethylester**

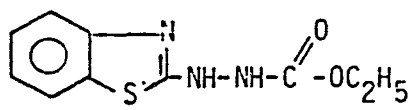

3.4 g (20 mmol) 2-Chlorbenzothiazol werden mit 2,1 g (20 mmol) Hydrazincarbonsäureethylester und 1,4 g (20 mmol) Triethylamin in 10 ml 99,7 %-igem Ethanol 12 Stunden bei Rückflußtemperatur umgesetzt. Nach dem Einengen der Reaktionslösung wird der Rückstand in Methylenchlorid aufgenommen, die organische Phase mit Wasser neutralgewaschen und über Natriumsulfat getrocknet. Nach dem Einengen der organischen Phase wird der Rückstand mit Ether gefällt.

Farblose Kristalle vom Schmelzpunkt 177 - 178 °C

Ausbeute: 0,3 g (6,4 % d.Th.)

Rf = 0,6 ($CH_2Cl_2$ / MeOH 9 : 1)

$C_{10}H_{11}N_3O_2S$ (237)

$^1$H-NMR-Spektrum: (d$_6$-DMSO, TMS als interner Standard)

$\delta$ = 1.23 (t) ($CH_3$-) 3 H,
4.13 (q) ($CH_2$-) 2 H,
6.93 - 7.87 (m) (Aromaten-H) 4 H,
9.67 (s) (N-H) (austauschbar mit $D_2O$) 1 H,
9.80 (s) (N-H) (austauschbar mit $D_2O$) 1 H ppm.

Beispiel 10

Herstellung von $N^2$-(Benzothiazol-2-yl)-hydrazincarbonsäure-tert. butylester

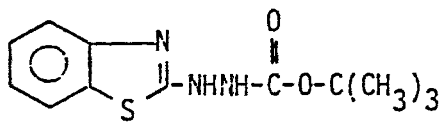

Die Herstellung erfolgt analog Beispiel 9 aus 6,8 g (40 mmol) 2-Chlorbenzothiazol und 5,3 g (40 mmol) Hydrazincarbonsäure-tert.-butylester

Farblose Kristalle vom Schmelzpunkt 160 - 163 °C

Ausbeute: 0.43 g (4.1 % d.Th.)

Rf = 0.69 ($CH_2Cl_2$ / MeOH 95:5)

$C_{12}H_{15}N_3O_2S$ (265)

$^1$H-NMR-Spektrum: ($d_6$-DMSO, TMS als interner Standard)

$\delta$ = 1.43 (s) (-C($CH_3$)$_3$) 9 H,
7.00 - 7.83 (m) (Aromaten-H) 4 H,
9.43 (s) (N-H) (austauschbar mit $D_2O$) 1 H,
9.66 (s) (N-H) (austauschbar mit $D_2O$) 1 H ppm.

# PATENTANSPRÜCHE

1. Arzneimittel, dadurch gekennzeichnet, daß es neben üblichen Hilfs- und Trägerstoffen als Wirkstoff ein Benzazolderivat der allgemeinen tautomeren Formeln I

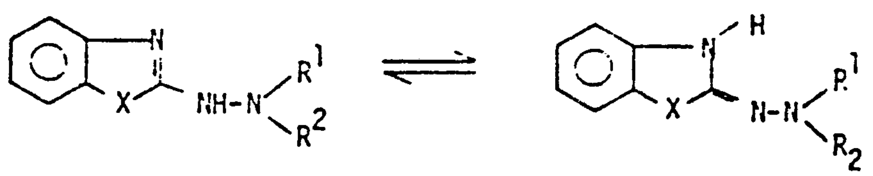

(I)

in denen X für ein Schwefel- oder Sauerstoffatom steht, $R^1$ ein Wasserstoffatom bedeutet und $R^2$ für eine Hydroxymethyl-, Formyl-, Carbniedrigalkoxy- oder eine Niedrigacylgruppe steht oder $R^1$ und $R^2$ zusammen für die Gruppierung

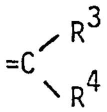 stehen, wobei $R^3$ und $R^4$ unabhängig voneinander ein Wasserstoffatom oder eine $C_1$-$C_6$-Alkylgruppe bedeuten, oder ein physiologisch annehmbares Salz davon enthält.

 

2. Verwendung eines Benzazolderivats der allgemeinen tautomeren Formeln I

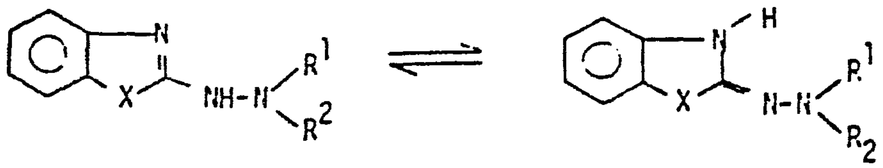 (I)

in denen X für ein Schwefel- oder Sauerstoffatom steht, $R^1$ ein Wasserstoffatom bedeutet und $R^2$ für eine Hydroxymethyl-, Formyl-, Carbniedrigalkoxy- oder eine Niedrigacylgruppe steht oder $R^1$ und $R^2$ zusammen für die Gruppierung

$=C\begin{smallmatrix} R^3 \\ R^4 \end{smallmatrix}$ stehen, wobei $R^3$ und $R^4$ unabhängig voneinander ein Wasserstoffatom oder eine $C_1$-$C_6$-Alkylgruppe bedeuten, sowie der physiologisch annehmbaren Salze davon zur Bekämpfung entzündlicher Erkrankungen.

3. Verwendung eines Benzazolderivats der allgemeinen tautomeren Formeln I

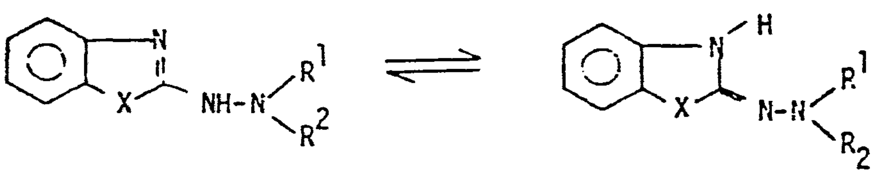 (I)

in denen X für ein Schwefel- oder Sauerstoffatom steht, $R^1$ ein Wasserstoffatom bedeutet und $R^2$ für eine Hydroxymethyl-, Formyl-, Carbniedrigalkoxy- oder eine Niedrigacylgruppe steht oder $R^1$ und $R^2$ zusammen für die Gruppierung

$=C\begin{smallmatrix} R^3 \\ R^4 \end{smallmatrix}$ stehen, wobei $R^3$ und $R^4$ unabhängig voneinander ein Wasserstoffatom oder eine $C_1$-$C_6$-Alkylgruppe bedeuten, sowie der physiologisch annehmbaren Salze davon zur Inhibierung des Lipoxygenase- und/oder Cyclooxygenasewegs des Arachidonsäurestoffwechsels.

4. Verwendung eines Benzazolderivats der allgemeinen tautomeren Formeln I

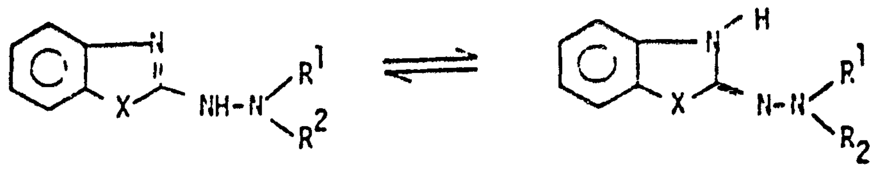

(I)

in denen X für ein Schwefel- oder Sauerstoffatom steht, $R^1$ ein Wasserstoffatom bedeutet und $R^2$ für eine Hydroxymethyl-, Formyl-, Carbniedrigalkoxy- oder eine Niedrigacylgruppe steht oder $R^1$ und $R^2$ zusammen für die Gruppierung

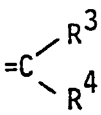 stehen, wobei $R^3$ und $R^4$ unabhängig voneinander ein Wasserstoffatom oder eine $C_1$-$C_6$-Alkylgruppe bedeuten, sowie der physiologisch annehmbaren Salze davon zur Herstellung von Arzneimitteln mit antiphlogistischer Wirkung.

## P A T E N T A N S P R O C H E

für den Vertragsstaat AT

1. Verfahren zur Herstellung eines Arzneimittels, dadurch g e k e n n z e i c h n e t , daß man ein Benzazolderivat der allgemeinen tautomeren Formeln I

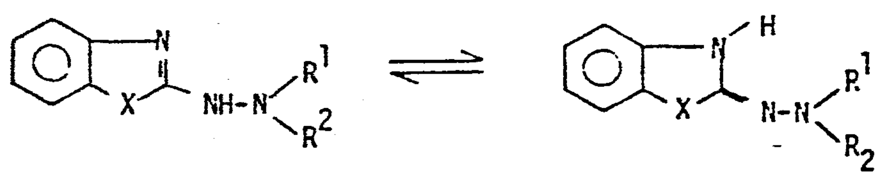

(I)

in denen X für ein Schwefel- oder Sauerstoffatom steht, $R^1$ ein Wasserstoffatom bedeutet und $R^2$ für eine Hydroxymethyl-, Formyl-, Carbniedrigalkoxy- oder eine Niedrigacylgruppe steht oder $R^1$ und $R^2$ zusammen für die Gruppierung

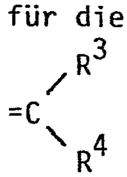

stehen, wobei $R^3$ und $R^4$ unabhängig voneinander ein Wasserstoffatom oder eine $C_1$-$C_6$-Alkylgruppe bedeuten, oder ein physiologisch annehmbares Salz davon mit einem üblichen Hilfs- oder Trägerstoff vermischt.

2. Verwendung eines Benzazolderivats der allgemeinen tautomeren Formeln I

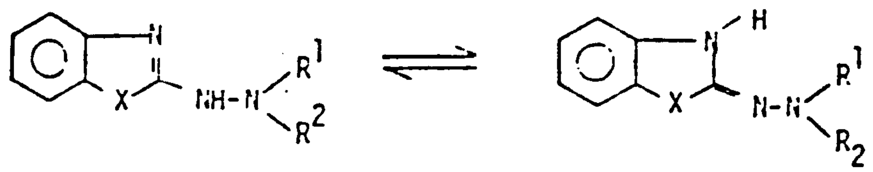
(I)

in denen X für ein Schwefel- oder Sauerstoffatom steht, $R^1$ ein Wasserstoffatom bedeutet und $R^2$ für eine Hydroxymethyl-, Formyl-, Carbniedrigalkoxy- oder eine Niedrigacylgruppe steht oder $R^1$ und $R^2$ zusammen für die Gruppierung

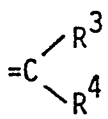 stehen, wobei $R^3$ und $R^4$ unabhängig voneinander ein Wasserstoffatom oder eine $C_1$-$C_6$-Alkylgruppe bedeuten, sowie der physiologisch annehmbaren Salze davon zur Bekämpfung entzündlicher Erkrankungen.

3. Verwendung eines Benzazolderivats der allgemeinen tautomeren Formeln I

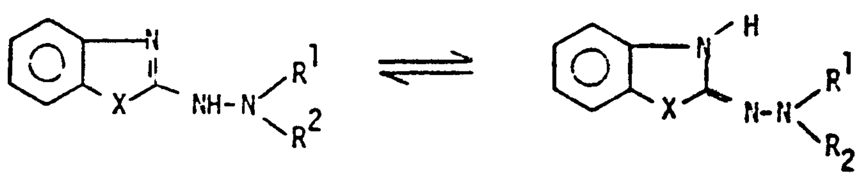
(I)

in denen X für ein Schwefel- oder Sauerstoffatom steht, $R^1$ ein Wasserstoffatom bedeutet und $R^2$ für eine Hydroxymethyl-, Formyl-, Carbniedrigalkoxy- oder eine Niedrigacylgruppe steht oder $R^1$ und $R^2$ zusammen für die Gruppierung

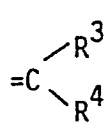 stehen, wobei $R^3$ und $R^4$ unabhängig voneinander ein Wasserstoffatom oder eine $C_1$-$C_6$-Alkylgruppe bedeuten, sowie der physiologisch annehmbaren Salze davon zur Inhibierung des Lipoxygenase- und/oder Cyclooxygenasewegs des Arachidonsäurestoffwechsels.

4. Verwendung eines Benzazolderivats der allgemeinen tautomeren Formeln I

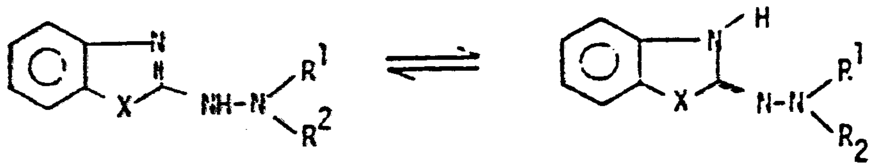 (I)

in denen X für ein Schwefel- oder Sauerstoffatom steht, $R^1$ ein Wasserstoffatom bedeutet und $R^2$ für eine Hydroxymethyl-, Formyl-, Carbniedrigalkoxy- oder eine Niedrigacylgruppe steht oder $R^1$ und $R^2$ zusammen für die Gruppierung

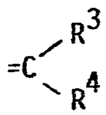 stehen, wobei $R^3$ und $R^4$ unabhängig voneinander ein Wasserstoffatom oder eine $C_1$-$C_6$-Alkylgruppe bedeuten, sowie der physiologisch annehmbaren Salze davon zur Herstellung von Arzneimitteln mit antiphlogistischer Wirkung.